**Europäisches Patentamt**

**European Patent Office**

**Office européen des brevets**

(19)

(11) Numéro de publication: **0 005 663**
**B1**

# FASCICULE DE BREVET EUROPEEN

(12)

(45) Date de publication du fascicule du brevet:
**17.08.83**

(51) Int. Cl.³: **A 61 N 1/42**

(21) Numéro de dépôt: **79400295.6**

(22) Date de dépôt: **10.05.79**

(54) **Appareil de traitement d'un substratum vivant.**

(30) Priorité: **17.05.78 FR 7814615**

(43) Date de publication de la demande:
**28.11.79 Bulletin 79/24**

(45) Mention de la délivrance du brevet:
**17.08.83 Bulletin 83/33**

(84) Etats contractants désignés:
**AT BE CH DE GB IT LU NL SE**

(56) Documents cités:
**DE-A-2 116 869**
**DE-A-2 353 959**
**DE-A-2 655 723**
**FR-A-1 573 153**
**FR-A-2 210 420**
**FR-A-2 291 773**
**FR-A-2 365 911**
**FR-A-2 370 483**

(73) Titulaire: **Constantinescu, Dan, 60, rue des Mathurins,
F-75008 Paris (FR)**
Titulaire: **Senez, Daniel, 9, rue Robert Vivier,
F-37200 Tours 03 (FR)**
Titulaire: **Senez, Michel, 4bis, rue du Bouvalot,
F-59283 Moncheaux (FR)**

(72) Inventeur: **Constantinescu, Dan, 60, rue des Mathurins,
F-75008 Paris (FR)**

(74) Mandataire: **Robert, Jean-Pierre et al, CABINET BEAU
DE LOMENIE 55, rue d'Amsterdam, F-75008 Paris (FR)**

Appareil de traitement d'un substratum vivant

La présente invention concerne un appareil de traitement d'un substratum vivant selon lequel des signaux magnétiques compatibles avec l'organisation informatique et l'autorégulation des organismes vivants sont émis de manière à être reçus par l'organisme et entraîner des réponses d'équilibration et de dynamisation fonctionnelles.

L'électromagnétothérapie dans ses divers aspects a considéré jusqu'à présent son action sur l'organisme vivant comme une action physique directe. Ainsi, l'électrothérapie de haute fréquence et l'électromagnétothérapie classique basent leur action sur le réchauffement en profondeur des tissus dû à l'absorption de l'énergie électromagnétique qui est convertie en chaleur selon la loi de Joule. L'électromagnétothérapie de haute fréquence pulsée invoque la conversion par mécanisme piezoélectrique, au niveau des structures cristallines des tissus pour expliquer ses effets biologiques athermiques. L'électrothérapie de basse fréquence, comme l'électromagnétothérapie de basse fréquence, invoque des actions d'inductions et de dissociations ioniques au niveau de tissus vivants.

Il faut considérer un autre mode d'action des champs électromagnétiques sur l'organisme vivant qui est d'ordre informationnel, agissant sur les phénomènes d'autorégulation des systèmes vivants dans leurs tendances à l'équilibration et à l'adaptation.

La récente biocybernétique a mis en évidence des systèmes régulateurs fonctionnant à l'aide de signaux sur tous les paliers du fonctionnement de l'organisme, du génome au comportement, comme caractères les plus généraux de l'organisation vitale.

La biochimie quantique montre que la structure électronique des macromolécules des composés essentiels qui forment l'essence dynamique de la matière vivante est celle de la décocalisation électronique.

Cette particularité de structure assure à la molécule vivante sa grande résistivité aux rayonnements vivants ionisants et en même temps, la grande polarisabilité, la mobilité et la fluidité du nuage électronique des molécules conjuguées sont autant de possibilités pour la transmission rapide des perturbations qui, en langage biologique, peuvent signifier un ordre ou un avertissement.

Des études récentes de biologie et de médecine énergétique ont mis en évidence l'existence de champs électromagnétiques organiques et en particulier leur rôle dans le système lent de contrôle, dans la détermination des niveaux d'activité fonctionnelle et de leurs rapports avec les micropulsations électromagnétiques externes.

Les études de biométérologie ont mis en évidence le rôle des fluctuations électromagnétiques de l'environnement naturel dans le spectre des basses et très basses fréquences à côté des micropulsations magnétiques terrestres pour le comportement des êtres vivants. On peut penser que ces fluctuations possèdent des caractères informationnels biologiques.

Dans ce cadre, l'invention a donc pour objet un appareil pour émettre vers un corps vivant des signaux susceptibles de posséder une signification informatique compatible avec ce qui pourrait être l'organisation informatique du substratum.

L'appareil pour émettre lesdits signaux comporte une tête d'émission pourvue d'au moins trois pièces polaires non alignées dont deux sont homonymes et une hétéronyme par rapport aux deux autres, lesdites pièces étant pourvues d'enroulements susceptibles d'être groupés électroniquement par paire et reliés à un générateur électrique de fréquence réglable entre 40 et 80 KHz, par l'intermédiaire d'un dispositif de filtrage des ondes électriques émises pour n'en sélectionner qu'un nombre déterminé réglable, de manière à former un train d'ondes, d'un dispositif de réglage de l'espacement dans le temps des trains d'ondes successifs entre 1 et 600 trains par seconde, et d'un dispositif logique préréglable pour assurer de manière prédéterminée les groupements par paire susdits au passage de chaque onde d'un train d'ondes. Ainsi, chaque signal est constitué par une succession de champs magnétiques d'orientations différentes dont la puissance est faible et la fréquence de l'ordre de la très basse fréquence radio. Il s'ensuit que le signal possède une forme dynamique dont l'expérience montre la grande importance et dont l'effet est en rapport vraisemblable avec le caractère anisotrope des molécules conjuguées du substratum vivant du point de vue magnétique.

Enfin, la répétition des alternances d'orientation constitue également une caractéristique de forme du signal qui assure à ce dernier un caractère systématique permettant d'être distingué par l'organisme de signaux aléatoires ou fortuits.

L'invention sera mieux comprise au cours de la description donnée ci-après à titre d'exemple purement indicatif et non limitatif qui permettra d'en dégager les avantages et les caractéristiques secondaires.

Il sera fait référence aux dessins annexés dans lesquels:

La figure 1 illustre schématiquement, un appareil selon l'invention,

Les figures 2 et 3, illustrent des variantes possibles d'une tête d'émission à trois pièces polaires,

Les figures 4 et 5 illustrent deux autres modes de réalisation de tête d'émission.

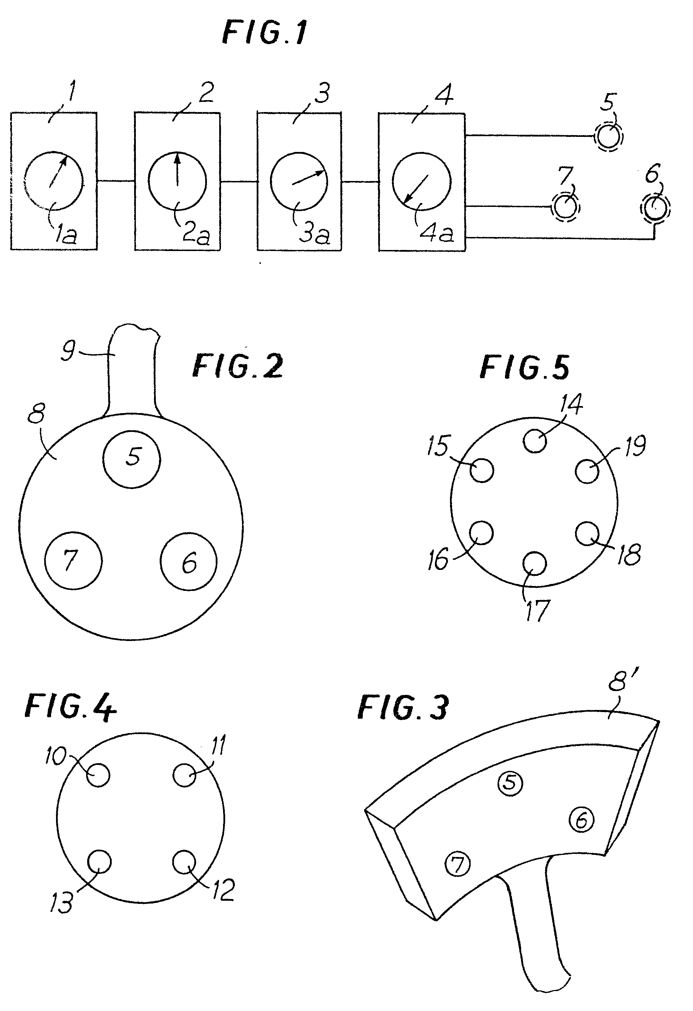

En se reportant tout d'abord à la figure 1, on voit un générateur de fréquence 1, susceptible d'émettre un courant de fréquence réglable par

1a entre 40 et 80 KHz. Ce courant est dirigé dans un dispositif 2 qui filtre un nombre prédéterminé d'ondes dont la valeur peut être affichée en 2a. Les ondes sélectionnées forment des trains d'ondes sortant du dispositif 2 à une fréquence fixe supérieure à 600 Hz. Un second appareil de filtrage 3, permet de régler par 3a, l'espacement dans le temps des trains d'ondes consécutifs émis en sortie du dispositif 2 depuis 1, jusqu'à 600 trains par seconde. De même, on peut incorporer de manière connue à l'ensemble ci-dessus, un dispositif permettant de transformer chaque onde en un signal électrique rectangulaire.

Ainsi, à la sortie du dispositif 3, on émet des trains d'ondes (ou d'impulsions rectangulaires), chaque train possédant un nombre déterminé d'ondes lesquelles se suivent à une fréquence également déterminée, chaque train étant séparé du suivant d'un temps également déterminé. Par exemple, si l'on affiche par 1a une fréquence de 80 KHz par 2a douze ondes et par 3a 100 Hz, en sortie de l'appareil 3, on obiendra une succession de trains d'ondes comprenant chacun douze ondes (ou impulsions rectangulaires) s'écoulant pendant 150 microsecondes, chaque train d'ondes étant émis tous les centièmes de secondes.

Une telle succession de trains d'ondes est ensuite dirigée sur des pièces polaires 5, 6 et 7 et plus précisément dans les enroulements de ces dernières. Ces enroulements sont tels que par exemple, les pièces polaires 5 et 7 sont homonymes et la pièce 6 est hétéronyme par rapport aux deux autres. Un dispositif logique 4 permet de réaliser des groupements par paire desdits enroulements selon un ordre prédéterminé par affichage modifiable (4a) afin que chaque onde d'un train d'ondes reçu à l'entrée du dispositif logique, soit dirigée sur un couple de pièces pôlaires, de manière à créer une impulsion magnétique dont la direction dépendra de l'homonymie ou de l'hétéronymie des pièces couplées.

Si par exemple, l'on affiche par 4a le couplage successif des enroulements des pièces 7 et 6, puis des pièces 7 et 5, puis des pièces 7 et 6, la première onde d'un train de douze ondes provoquera la création d'une impulsion magnétique circulant entre les pièces 7 et 6. L'onde suivante provoquera un champ magnétique entre les pièces 7 et 5, c'est-à-dire, puisque les pièces 7 et 5 sont homonymes circumant perpendiculairement au plan qui les contient. La troisième onde établira une impulsion semblable à celle créée par la première. Ce cycle se reproduira ainsi au passage des trois autres groupes de trois ondes que comporte encore le train d'ondes. Le signal magnétique sortant des pièces pôlaires est donc constitué par quatre répétitions d'un sous signal de base dont la forme dépend des couplages affichés par 4a dans le dispositif logique 3.

Les pièces polaires 5, 6 et 7 sont portées de manière non alignée, par une tête d'émission 8, telle que représentée sur la figure 2. Cette tête peut être plane ou de forme quelconque (concave telle qu'en 8' sur la figure 3), reliée par un câble souple 9 à la sortie de l'appareil référencé 1 à 4 à la figure 1. On voit bien que lorsqu'on applique la face visible sur la figure 2 de la tête 8 sur le corps d'un patient, les impulsions magnétiques émises par les pièces 5 à 7 s'étendent dans les tissus vivants sensiblement parallèlement à la face susdite, donc peu en profondeur, soit perpendiculairement à cette dernière, donc plus en profondeur ce, dans une séquence déterminée répétée au moins trois fois pour un seul train d'ondes pendant un temps compris entre 100 et 200 microsecondes et réitérées à une fréquence égale à la fréquence de succession des trains d'ondes.

On peut bien entendu réaliser des couplages différents et dans un ordre différent. Ainsi, en couplant tour à tour 6-5 puis 5-7, puis 7-1 ce, quatre fois de suite, on obtient un signal légèrement différent de celui précédemment décrit en ce sens que les champs magnétiques consécutifs issus de deux pôles homonymes sont de directions différentes.

Sur la figure 4, on a représenté schématiquement une tête d'émission à quatre pièces polaires 10, 11, 12, 13, situées au sommet d'un carré dont 10, 11 et 12 sont de pôlairité semblable et 13 de polarité contraire. L'expérience montre qu'un signal issu de trois répétitions au moins des couplages 10-12, 11-13 est d'une très grande efficacité.

Sur la figure 5 enfin, les pièces pôlaires 14, 15, 16, 17, 18, 19 sont placées au sommet d'un hexagone régulier. Les pôles 16 et 18 non consécutifs sont de pôlarité semblable et contraire à celle des quatre autres pôles. L'expérience a montré que les signaux les plus efficaces susceptibles d'être émis par une telle tête sont issus d'une répétition (au moins trois fois), soit des couplages 14-16-18, 15-17-19, soit des couplages 14-17, 15-18, 16-19. La tête de la figure 5 peut avantageusement être utilisée — en sélectionnant une partie seulement des pôles 14 à 19 — somme une tête à trois pôles ou une téte à quatre pôles.

Les trois types de tête ci-dessus décrits constituent des types fondamentaux; une tête quelconque à plus de six pôles constitue une combinaison de ces trois types.

L'ensemble des impulsions magnétiques émises lors du passage d'un train d'ondes constitue un signal de forme bien déterminée. Ce signal agit comme un langage qui s'intègre dans l'organisation informatique du substratum vivant qui le reçoit. Il en résulte des modifications d'ordre fonctionnel biologique. La forme géométrique du signal pouvant être modifiée, au moyen de la séquence de couplage affichée, de même que son intensité et sa fréquence, on obtient ainsi des ensembles significatifs pour déterminer des actions physiologiques susceptibles d'aider les processus naturels de défense et de guérison de l'organisme ainsi que la réadaptation fonctionnelle.

Ainsi, on a constaté qu'un signal émis à une fréquence entre 4 et 16 Hz et à très faible intensité, permet d'augmenter le seuil de sensibilité d'un patient (suppression d'une douleur locale). En revanche, entre 400 et 600 Hz, avec des intensités plus fortes, on facilite la conduction nerveuse et on dynamise les réactions de défense de l'organisme. Enfin, entre 80 et 120 Hz avec des puissances moyennes, on agit favorablement sur l'équilibration métabolique du substrat traité.

Le dispositif de l'invention, par le fait qu'il produit des signaux (l'induction magnétique pouvant être réglée entre 0,0001 et 0,01 tesla) ayant une énergie faible n'ayant pas d'action physique directe sur l'organisme ne provoque aucune modification irréversible du substratum vivant (destruction anatomique, ou même désorganisation des mécanismes de régulation). Son champ d'action se situe dans le domaine de la biologie électronique et de la biocybernétique, le signal qu'il émet étant compatible avec les signaux de l'organisation informatique du substratum et possédant pour lui une signification biologique.

On pourra prévoir dans certaines variantes non représentées, des têtes d'émission soit en matériau souple permettant de s'adapter au moins approximativement aux contours de la partie — notamment du corps humain — à traiter, soit en deux ou plusieurs parties articulées, chacune d'elles portant des pôles susceptibles d'être toujours couplés ensemble (tels que les pôles 10-12 et 11-13 de la figure 3 ou 14-16-18 et 15-17-19 de la figure 6 pour un type de couplage).

Le dispositif d'émission revendiqué trouve une application intéressante dans le domaine des appareils médicaux et paramédicaux.

**Revendications**

1. Dispositif d'émission d'un signal magnétique destiné à être utilisé dans une méthode de traitement par magnétothérapie d'un corps vivant, comportant une tête d'émission (8) équipée d'au moins trois pièces pôlaires (5, 6, 7) contenues de manière non alignée, dans une surface à placer en regard du corps vivant pourvues d'enroulements pour définir deux pièces polaires homonymes (5, 7) et une hétéronyme (6) par rapport aux deux autres lors de la circulation dans ces enroulements d'un courant électrique, caractérisé en ce qu'il comprend un générateur électrique (1) de fréquence réglable entre 40 et 80 KHz, relié à ladite tête d'émission (8) par l'intermédiaire d'un dispositif de filtrage (2) des ondes électriques émises pour n'en sélectionner qu'un nombre déterminé réglable, de manière à former un train d'ondes; d'un dispositif (3) de réglage de l'espacement dans le temps des trains d'ondes successifs entre 1 et 600 trains par seconde, et d'un dispositif logique (4) préréglable pour assurer de manière prédéterminée, les groupements par paire successifs desdits enroulements au passage de chaque onde d'un train d'ondes.

2. Dispositif selon la revendication 1, caractérisé en ce que la tête d'émission comporte quatre pièces pôlaires (10, 11, 12, 13) disposées au sommet d'un carré, l'une d'elles étant de pôlarité opposée à celle des trois autres (10, 11, 12).

3. Dispositif selon la revendication 1, caractérisé en ce que la tête d'émission comporte six pièces pôlaires (14–18) disposées au sommet d'un hexagone régulier deux d'entre elles (16, 18) non consécutives étant de même pôlarité et contraire à celle des quatre autres.

4. Dispositif selon l'une quelconque des revendications 1 à 3, caractérisé en ce que la surface susdite est plane.

5. Dispositif selon l'une quelconque des revendications 1 à 3, caractérisé en ce que la surface susdite est non plane.

6. Dispositif selon la revendication 5, caractérisé en ce que la surface susdite est déformable.

**Patentansprüche**

1. Vorrichtung zur Sendung eines magnetischen Signals zur Verwendung in einem therapeutischen Verfahren durch Magnetotherapie eines lebendigen Körpers, welche einen Sendekopf (8) mit wenigstens drei nicht ausgefluchteten Polstücken (5, 6, 7), die in einem gegenüber dem lebendigen Körper anzuordnenden Bereich enthalten sind, und Wicklungen zur Bestimmung von zwei gleichnamigen (5, 7) Polstücken und von einem gegenüber den beiden anderen ungleichnamigen Polstück (6) beim Durchgang eines elektrischen Signals in diesen Wicklungen aufweisen, aufweist, dadurch gekennzeichnet, daß sie einen zwischen 40 und 80 kHz regelbaren elektrischen Generator (1) aufweist, der mit dem genannten Sendekopf (8) über eine Vorrichtung (2) zur Filterung der gesendeten elektrischen Wellen verbunden ist, um davon nur eine regelbare gegebene Anzahl derart auszuwählen, daß ein Wellenzug gebildet wird; über eine Vorrichtung (3) zur Regelung des Zeitabstandes der aufeinanderfolgenden Wellenzüge von 1 bis 600 Zügen/s und über eine vorregelbare logische Vorrichtung (4), um die in Paaren erfolgenden Gruppierungen der genannten Wicklungen beim Durchgang jeder Welle eines Wellenzuges in vorgegebener Weise zu gewährleisten.

2. Vorrichtung nach Anspruch 1, dadurch gekennzeichnet, daß der Sendekopf an den Ecken eines Quadrats angeordnete Polstücke (10, 11, 12, 13) aufweist, von denen eines eine gegenüber den drei anderen (10, 11, 12) entgegengesetzte Polarität aufweist.

3. Vorrichtung nach Anspruch 1, dadurch gekennzeichnet, daß der Sendekopf an den Ecken eines regelmäßigen Sechsecks angeordnete Polstücke (14–18) aufweist, von denen zwei (16, 18) nicht aufeinanderfolgende dieselbe und gegenüber den vier anderen die entgegengesetzte Polarität aufweisen.

4. Vorrichtung nach einem der Ansprüche 1 bis 3, dadurch gekennzeichnet, daß der obengenannte Bereich eben ist.

5. Vorrichtung nach einem der Ansprüche 1 bis 3, dadurch gekennzeichnet, daß der obengenannte Bereich nicht eben ist.

6. Vorrichtung nach Anspruch 5, dadurch gekennzeichnet, daß der obengenannte Bereich verformbar ist.

**Claims**

1. An apparatus for emitting a magnetic signal useful in a method for treating a living body by magnetotherapy, said apparatus comprising an emission head (8) provided with at least three non-aligned poles pieces (5, 6, 7) contained in a surface to be placed in front of said living body and comprising coils to define two homonymous poles pieces (5, 7) and one pole piece (6) heteronymous with respect to the other two when said coils are electrically energized characterized in that said apparatus comprises — an eletric generator of frequency (1) adjustable between 40 and 80 kHz connected to the said emission head (8) via a filtration device (2) for filtering the emitted electric waves so as to select only a predetermined adjustable number thereof and form a wave train; — a device (3) for controlling the time interval between the successive wave trains at between 1 and 600 trains per second; — and a preadjustable logical device (4) to ensure, in a predetermined manner the successive pairing-up of said coils when each wave of a wave train passes.

2. An apparatus according to claim 1, characterized in that the emission head comprises four pole pieces (10, 11, 12, 13) placed at the top of a square, the polarity of one of which is reverse to that of the other three (10, 11, 12).

3. An apparatus according to claim 1, characterized in that the emission head comprises six pole pieces (14−19) placed at the top of a regular hexagon, two of which (16, 18) are non-consecutive and of similar polarity whilst being of reverse polarity with respect to the other four.

4. An apparatus according to claims 1, 2 or 3, characterized in that the pole pieces are contained in a plane surface.

5. An apparatus according to any one of claims 1 to 3, characterized in that the pole pieces are carried by a non-plane surface.

6. An apparatus according to claim 5, characterized in that said surface is deformable.

# FIG.1

1   2   3   4   5   7   6

1a   2a   3a   4a

9

8

# FIG.2

5

7   6

# FIG.5

14
15   19
16   18
17

# FIG.4

10   11
13   12

# FIG.3

8'

5
6
7